# EUROPEAN PATENT APPLICATION

(11) **EP 4 597 111 A1**
(43) Date of publication of application: **06.08.2025**
(21) Application number: 24869432.5
(22) Date of filing: 23.05.2024
(51) Int. Cl.: G01N 33/574, G01N 30/02, G01N 30/72

(54) **USE OF METABOLIC MARKER FOR DIAGNOSIS OF LUNG CANCER STAGING AND KIT**

(30) Priority: 13.12.2023 CN 202311708074
(71) Applicant: Harbin Metanotitia Inc., Harbin, Heilongjiang 150029 (CN)
(72) Inventor: LI, Yan, Harbin, Heilongjiang 150029 (CN); WEN, He, Harbin, Heilongjiang 150029 (CN); WANG, Jiaojiao, Harbin, Heilongjiang 150029 (CN); LIU, Li, Harbin, Heilongjiang 150029 (CN); ZHOU, Xinying, Harbin, Heilongjiang 150029 (CN); HUANG, Guihong, Harbin, Heilongjiang 150029 (CN); WEI, Qingyan, Harbin, Heilongjiang 150029 (CN); HU, Junyuan, Harbin, Heilongjiang 150029 (CN)
(74) Representative: Rauch, Udo
(86) International application number: PCT/CN2024/094913
(87) International publication number: WO 2025/123592

(57) **Abstract**

Use of metabolic markers for diagnosing stages of lung cancer and a kit are provided. Use of metabolic markers for diagnosing stages of lung cancer and one or more of a detection reagent for the metabolic markers for diagnosing stages of lung cancer in preparation of a kit for diagnosing the stages of lung cancer are provided. A kit for diagnosing stages of lung cancer is provided, including metabolic markers for diagnosing stages of lung cancer and one or more of a detection reagent for the metabolic markers for diagnosing stages of lung cancer.

## Description

### RELATED APPLICATION

The present application claims priority to Chinese Patent Application No. 202311708074.6 filed on December 13, 2023 and entitled "USE OF METABOLIC MARKERS FOR DIAGNOSING STAGES OF LUNG CANCER AND KIT", which is incorporated herein by reference in its entirety.

### TECHNICAL FIELD

The present disclosure relates to the field of biomedical technology, and in particular to use of metabolic markers for diagnosing stages of lung cancer and a kit.

### BACKGROUND

Lung cancer is one of the most common cancers with a high mortality rate worldwide, showing a 5-year survival rate of only 54%. Accurate diagnosis of the lung cancer is crucial for improving patients' survival rates. However, currently only 15% of lung cancer patients can be detected at an early stage. Most patients are diagnosed at an advanced stage when the disease becomes incurable, which is also the primary cause of death in most lung cancer patients. The objectives and methods of treatment for lung cancer vary by stages. Early-stage treatment primarily aims to surgically remove the tumor, prevent the spread and metastasis of cancer cells, and preserve as much normal lung tissue as possible. In the mid-stage, the chances of surgery are relatively reduced, and comprehensive treatment is generally adopted, including chemotherapy, radiotherapy, targeted drug therapy, immunotherapy, and traditional Chinese medicine treatment. For patients with advanced lung cancer, the main goals of treatment are to relieve symptoms, improve quality of life, and prolong survival. Therefore, accurate diagnosis of stages of lung cancer can provide an important reference for standardized and individualized treatment, which helps to improve treatment outcomes, improve quality of life, and prolong survival of patients.

At present, the lung cancer screening mainly includes techniques such as low-dose spiral computed tomography (LDCT), bronchoscopy, and cytological specimen detection. LDCT is mainly used for lung cancer screening in high-risk groups and has achieved some success to a certain extent. However, LDCT promotion is limited by high cost, increased false positive rate, and potential radiation exposure risks. Bronchoscopy, cytological specimen detection, and pathological diagnosis are currently the main approaches for diagnosing lung cancer. However, these methods face challenges, particularly in accurately diagnosing tumors located at the lung's periphery or hidden tumors. Bronchoscopy sometimes cannot fully observe the edge area of the lungs, while cytological specimen detection may not be sensitive enough for certain types of tumors. Pathological diagnosis is difficult for special types of lung cancer, such as neuroendocrine cancer, and multiple biopsies are required to confirm the diagnosis, thus increasing pain, time, and cost of the patient. In view of this, it is urgently needed for more cost-effective, accurate, and non-invasive or minimally invasive screening and diagnostic methods for lung cancer.

Metabolomics studies reveal biological processes related to metabolic changes by analyzing variations in metabolites produced by the body in response to disease or specific physiological conditions. By studying these metabolites, it is possible to better tailor treatment strategies to individual patients. In the diagnosis of lung cancer, existing lung cancer diagnostic markers such as carcinoembryonic antigen (CEA), neuron-specific enolase (NSE), squamous cell carcinoma antigen (SCC), carbohydrate antigen 125 (CA125), and a soluble fragment of cytokeratin 19 (CYFRA21-1), have certain clinical application value but show limitations. These markers are primarily used to support diagnosis and monitor disease progression of lung cancer, but they are insufficient for accurately predicting the cancer stage. The definitive staging of lung cancer still relies on pathological evaluation. Therefore, there is an urgent need for the development of more specific and sensitive tumor markers to enhance both the diagnosis and treatment of lung cancer. This need is especially critical when addressing challenges such as atypical histological patterns and difficulties in differential diagnosis during lung cancer staging. The development of novel tumor markers can significantly improve the accuracy of lung cancer diagnosis and staging, providing patients with more precise and effective treatment options and enabling more personalized approaches to care. This has profound clinical implications for improving therapeutic outcomes and boosting survival rates among lung cancer patients.

### SUMMARY

A purpose of the embodiment in the present disclosure is to provide use of metabolic markers for diagnosing stages of lung cancer and a kit. A technical solution of the present disclosure is as follows:
The present disclosure provides use of metabolic markers for diagnosing stages of lung cancer and/or a detection reagent thereof in preparation of a kit for diagnosing the stages of lung cancer; where
the metabolic markers for diagnosing stages of lung cancer are selected from the group consisting of dehydroisoandrosterone-3-sulfate, 4-hydroxy-1-(3-pyridyl)-1-butanone, L-leucyl-L-leucine, L-octanoylcarnitine, phosphatidylcholine 34:3e, acyl carnitine 10:0, N-formyl-L-methionine, 5'-methylthioadenosine, phosphatidylcholine 36:3e, fatty acid 22:6, L-tryptophyl-L-phenylalanine, acyl carnitine 10:1, hypoxanthine, L-alanyl-L-aspartic acid, iminodiacetic acid, betaine, choline, O-palmitoyl-L-carnitine, and L-glutamic acid.

Alternatively, the detection reagent detects the metabolic markers for diagnosing stages of lung cancer through liquid chromatography-mass spectrometry (LC-MS).

Alternatively, a sample detected by the detection reagent includes a blood sample.

Alternatively, the blood sample includes the plasma.

Alternatively, a process of detecting the metabolic markers for diagnosing stages of lung cancer by the detection reagent through the LC-MS includes the following steps:
separating an organic phase and an aqueous phase from the plasma; and
detecting the organic phase and the aqueous phase through the LC-MS to detect the metabolic markers for diagnosing stages of lung cancer.

Alternatively, LC for detecting the organic phase includes:
a C8 chromatographic column as a stationary phase;
a mobile phase including a mobile phase A and a mobile phase B, where the mobile phase A is an aqueous solution containing 0.08% weight per volume (w/v) to 0.12% (w/v) of acetic acid and 0.08% (w/v) to 0.12% (w/v) of ammonium acetate; and the mobile phase B is a mixed solution of acetonitrile and isopropanol at a volume ratio of (6.5-7.5):(2.5-3.5) containing 0.08% (w/v) to 0.12% (w/v) of the acetic acid and 0.8% (w/v) to 1.2% (w/v) of the ammonium acetate; and
an elution method includes gradient elution, and a procedure of the gradient elution includes:
at 0 min to 12 min, the mobile phase B has a volume percentage increasing from 55% to 89%; and
at 12 min to 19.5 min, the mobile phase B has a volume percentage increasing from 89% to 100%.

Alternatively, LC for detecting the aqueous phase includes:
a T3 chromatographic column as a stationary phase;
a mobile phase including a mobile phase A and a mobile phase B, where the mobile phase A is an aqueous solution containing 0.08% (w/v) to 0.12% (w/v) of formic acid; and the mobile phase B is an acetonitrile solution containing 0.08% (w/v) to 0.12% (w/v) of the formic acid; and
an elution method includes gradient elution, and a procedure of the gradient elution includes:
at 0 min to 13 min, the mobile phase B has a volume percentage increasing from 1% to 70%; and
at 13 min to 18 min, the mobile phase B has a volume percentage increasing from 70% to 99%.

Alternatively, MS for detecting includes:
conducting acquisition in two modes of a Full MS mode and a Full MS/dd-MS2 mode, where the two modes each include positive and negative modes;
the Full MS mode has a resolution of 35,000 to 70,000, a scan range of 100 m/z to 1,500 m/z, an automatic gain control (AGC) of 3E+6, and a Maximum injection time (Maximum IT) of 150 ms to 250 ms; and
the Full MS/dd-MS2 mode has a secondary MS resolution of 17,500 to 35,000, a quadrupole rod window of 1.2 m/z to 1.6 m/z, an AGC of 1E+5, a maximum ion injection time of 45 ms to 55 ms, and a high-energy collision dissociation (HCD) relative collision energy of 10 eV to 45 eV

Alternatively, a process of separating the organic phase and the aqueous phase from the plasma includes:
extracting the plasma with a solvent 1 to obtain an extract; where the solvent 1 includes methyl tert-butyl ether (MTBE) and methanol; and
extracting the extract with a solvent 2, and conducting layering to collect an obtained upper layer to obtain the organic phase and collect an obtained lower layer to obtain the aqueous phase.

The examples of present disclosure further provides a kit for diagnosing stages of lung cancer, including the metabolic markers for diagnosing stages of lung cancer and/or the detection reagent thereof.

Details of one or more examples of the present disclosure are set forth in the description that follows, and other features, objectives, and advantages of the present disclosure will be apparent from the specification and its claims.

### BRIEF DESCRIPTION OF THE DRAWINGS

To explain the technical solutions in examples of the present disclosure more clearly for those skilled in the art to understand the present disclosure and its beneficial effects, the drawings required in the examples will be described below in brief. Apparently, the drawings in the following description show merely some examples of this application, and other drawings may be derived from these drawings by those of ordinary skill in the art without creative efforts.
FIG. 1 shows a linear correlation analysis diagram of the dehydroisoandrosterone-3-sulfate;
FIG. 2 shows a linear correlation analysis diagram of the 4-hydroxy-1-(3-pyridyl)-1-butanone;
FIG. 3 shows a linear correlation analysis diagram of the L-leucyl-L-leucine;
FIG. 4 shows a linear correlation analysis diagram of the L-octanoylcarnitine;
FIG. 5 shows a linear correlation analysis diagram of the phosphatidylcholine 34:3e;
FIG. 6 shows a linear correlation analysis diagram of the acyl carnitine 10:0;
FIG. 7 shows a linear correlation analysis diagram of the N-formyl-L-methionine;
FIG. 8 shows a linear correlation analysis diagram of the 5'-methylthioadenosine;
FIG. 9 shows a linear correlation analysis diagram of the phosphatidylcholine 36:3e;
FIG. 10 shows a linear correlation analysis diagram of the fatty acid 22:6;
FIG. 11 shows a linear correlation analysis diagram of the L-tryptophyl-L-phenylalanine;
FIG. 12 shows a linear correlation analysis diagram of the acyl carnitine 10:1;
FIG. 13 shows a linear correlation analysis diagram of the hypoxanthine;
FIG. 14 shows a linear correlation analysis diagram of the L-alanyl-L-aspartic acid;
FIG. 15 shows a linear correlation analysis diagram of the iminodiacetic acid;
FIG. 16 shows a linear correlation analysis diagram of the betaine;
FIG. 17 shows a linear correlation analysis diagram of the choline;
FIG. 18 shows a linear correlation analysis diagram of the O-palmitoyl-L-carnitine;
FIG. 19 shows a linear correlation analysis diagram of the L-glutamic acid;
FIG. 20 shows the evaluation results of a receiver operating characteristic (ROC) curve of the lung cancer diagnosis model based on 19 metabolic markers;
FIG. 21 shows the analysis results of Mirco-averaged ROC curve for multi-class classification of a training set of 19 metabolic markers in stages of lung cancer;
FIG. 22 shows the analysis results of Micro-averaged ROC curve for multi-class classification of a test set of 19 metabolic markers in stages of lung cancer;
FIG. 23 shows the analysis results of the ROC curves of a validation set of 19 metabolic markers in the lung cancer diagnosis; and
FIG. 24 shows the analysis results of Micro-averaged ROC curve for multi-class classification of a validation set of 19 metabolic markers in stages of lung cancer.

### DETAILED DESCRIPTION OF THE EMBODIMENTS

The goal of one or more examples of the present disclosure is to diagnose lung cancer and predict clinical stages through metabolic markers, provide more targeted guidance and reference for lung cancer prevention strategies and treatment plans, thereby reducing the mortality rate of lung cancer, and making positive contributions to improving patients' quality of life, reducing medical costs, and reducing the impact of lung cancer. Diagnosing lung cancer at an early stage allows for targeted treatment before the tumor spreads or worsens. The lung cancer metabolic markers explored in the examples can achieve lung cancer diagnosis and clinical staging prediction at one time. Compared with the traditional technical solutions, the technical solutions provided by one or more examples have the following advantages: the lung cancer metabolic markers selected in the examples have the advantages of non-invasiveness, high prediction accuracy, rapid acquisition of test results, early diagnosis, and clinical staging prediction. The metabolic markers are also helpful in monitoring the growth of tumors and changes in treatment effects, improving the chance of diagnosis of early stage, and reducing the risk of death caused by prognosis of advanced stage. The advantages of some examples in the present disclosure are as follows: (1) there is a large number of samples, increasing the richness and reliability of data. (2) In the lung cancer diagnosis model disclosed in the present disclosure, a maximum predicted value of the area under curve (AUC) is 0.968, which can show desirable prediction performance and accuracy. (3) The screening source process of the metabolic markers is more standardized and reliable, the screening process is clear, and the steps of the metabolic marker screening are clear, thus facilitating one-time lung cancer diagnosis and clinical staging prediction. (4) The selected metabolic markers are statistically significant. The selected metabolic markers have significant P values, which means that the changes in the selected metabolic markers are significantly different between lung cancer patients and healthy individuals, and can provide more accurate and reliable information for the diagnosis and prediction of lung cancer. (5) In the examples in the present disclosure, multi-class classification ROC analysis is conducted to more comprehensively explore the potential of metabolic markers in stages of lung cancer prediction, showing obvious advantages.

### A first aspect of the examples in the present disclosure

The present disclosure provides a metabolic marker for diagnosing stages of lung cancer and/or a detection reagent thereof in preparation of a kit for diagnosing the stages of lung cancer; where
the metabolic marker for diagnosing stages of lung cancer is selected from the group consisting of dehydroisoandrosterone-3-sulfate, 4-hydroxy-1-(3-pyridyl)-1-butanone, L-leucyl-L-leucine, L-octanoylcarnitine, phosphatidylcholine 34:3e, acyl carnitine 10:0, N-formyl-L-methionine, 5'-methylthioadenosine, phosphatidylcholine 36:3e, fatty acid 22:6, L-tryptophyl-L-phenylalanine, acyl carnitine 10:1, hypoxanthine, L-alanyl-L-aspartic acid, iminodiacetic acid, betaine, choline, O-palmitoyl-L-carnitine, and L-glutamic acid.

In the present disclosure, there is no particular limitation on the detection reagent. Alternatively, the detection reagent detects the metabolic marker for diagnosing stages of lung cancer through LC-MS.

In some embodiments, a sample detected by the detection reagent includes a blood sample. Alternatively, the blood sample is selected from the group consisting of the plasma and the serum. There is no particular limitation on a sample form of the blood sample, which may be, for example, a dried blood spot.

In some embodiments, a process of detecting the metabolic marker for diagnosing stages of lung cancer by the detection reagent through the LC-MS includes the following steps:
separating an organic phase and an aqueous phase from the plasma; and
detecting the organic phase and the aqueous phase through the LC-MS to detect the metabolic marker for diagnosing stages of lung cancer.

In some embodiments, LC for detecting the organic phase includes:
a C8 chromatographic column as a stationary phase;
a mobile phase including a mobile phase A and a mobile phase B, where the mobile phase A is an aqueous solution containing 0.08% weight per volume (w/v) to 0.12% (w/v) of acetic acid (such as 0.08%, 0.09%, 0.1%, 0.11%, and 0.12%) and 0.08% (w/v) to 0.12% (w/v) of ammonium acetate (such as 0.08%, 0.09%, 0.1%, 0.11%, and 0.12%); and the mobile phase B is a mixed solution of acetonitrile and isopropanol at a volume ratio of (6.5-7.5):(2.5-3.5) (such as 6.5:2.5, 6.5:3, 6.5:3.5, 7:2.5, 7:3, 7:3.5, 7.5:2.5, 7.5:3, and 7.5:3.5) containing 0.08% (w/v) to 0.12% (w/v) of the acetic acid (such as 0.08%, 0.09%, 0.1%, 0.11%, and 0.12%) and 0.8% (w/v) to 1.2% (w/v) of the ammonium acetate (such as 0.8%, 0.9%, 1%, 1.1%, and 1.2%); and
an elution method includes gradient elution, and a procedure of the gradient elution includes:
at 0 min to 12 min, the mobile phase B has a volume percentage increasing from 55% to 89%; and
at 12 min to 19.5 min, the mobile phase B has a volume percentage increasing from 89% to 100%.

In some embodiments, LC for detecting the aqueous phase includes:
a T3 chromatographic column as a stationary phase;
a mobile phase including a mobile phase A and a mobile phase B, where the mobile phase A is an aqueous solution containing 0.08% (w/v) to 0.12% (w/v) of formic acid; and the mobile phase B is an acetonitrile solution containing 0.08% (w/v) to 0.12% (w/v) of the formic acid; and
an elution method includes gradient elution, and a procedure of the gradient elution includes:
at 0 min to 13 min, the mobile phase B has a volume percentage increasing from 1% to 70%; and
at 13 min to 18 min, the mobile phase B has a volume percentage increasing from 70% to 99%.

In some embodiments, MS for detecting includes:
conducting acquisition in two modes of a Full MS mode and a Full MS/dd-MS2 mode, where the two modes each include positive and negative modes;
the Full MS mode has a resolution of 35,000 to 70,000, a scan range of 100 m/z to 1,500 m/z, an AGC of 3E+6, and a Maximum IT of 150 ms to 250 ms; and
the Full MS/dd-MS2 mode has a secondary MS resolution of 17,500 to 35,000, a quadrupole rod window of 1.2 m/z to 1.6 m/z, an AGC of 1E+5, a maximum ion injection time of 45 ms to 55 ms, and a HCD relative collision energy of 10 eV to 45 eV.

In some embodiments, a process of separating the organic phase and the aqueous phase from the plasma includes:
extracting the plasma with a solvent 1 to obtain an extract; where the solvent 1 includes MTBE and methanol; and
extracting the extract with a solvent 2, and conducting layering to collect an obtained upper layer to obtain the organic phase and collect an obtained lower layer to obtain the aqueous phase; where the solvent 2 includes methanol and water.

In some embodiments, the steps of extracting satisfy one or more of the following conditions:
(1) an extraction method includes: vortex;
(2) a dosage of the solvent 1 corresponding to per 100 µL of the plasma is 800 µL to 1,200 µL (e.g., 800, 850, 900, 950, 1,000, 1,050, 1,100, 1,150, and 1,200 µL); and
(3) the MTBE and the methanol in the solvent 1 is at a volume ratio of (2.5-3.5):1 (such as 2.5:1, 2.6:1, 2.7:1, 2.8:1, 2.9:1, 3.0:1, 3.1:1, 3.2:1, 3.3:1, 3.4:1, and 3.5:1).

In some embodiments, the steps of extracting further satisfy one or more of the following conditions:
(1) the methanol and the water in the solvent 2 is at a volume ratio of (2.5-3.5):1; and
(2) an extraction method includes: ultrasonic treatment, standing, and vortex.

In a second aspect of the examples in the present disclosure

The present disclosure further provides a kit for diagnosing stages of lung cancer, including the metabolic marker for diagnosing stages of lung cancer and/or the detection reagent thereof in the first aspect.

### In a third aspect of the examples in the present disclosure

The present disclosure further provides a method for diagnosing stages of lung cancer, including the following steps:
detecting a level of the metabolic marker for diagnosing stages of lung cancer defined in the first aspect in a sample isolated from a subject to be tested, and determining whether the subject to be tested suffers from lung cancer according to an obtained detection result.

In an example of the present disclosure, a process of determining whether the subject to be tested suffers from lung cancer according to the obtained detection result includes: determining whether the subject suffers from lung cancer, and determining a stage of the lung cancer if the subject suffers from the lung cancer.

In the third aspect, the definitions of "sample" and "detection reagent" used in the detection are the same as those in the first aspect.

The technical solutions of the present disclosure will be described in detail below with reference to examples. It should be understood that these examples are merely intended to describe the present application, rather than to limit the scope of the present application. For experimental methods in the following examples where specific conditions are not specified, reference should be made to the instructions given in the present disclosure, or according to experimental manuals or conventional conditions in the art, or according to conditions recommended by manufacturers or reference to experimental methods known in the art.

In the following specific examples, the measurement parameters of the raw material components may have slight deviations within the accuracy range of weighing unless otherwise specified. Parameters involving temperature and time allow for acceptable deviations due to the test accuracy or operating precision of the instrument.

### Example 1: Screening of differential metabolites in plasma of healthy control group and lung cancer group

### 1. Detection subjects and methods

(1) Written informed consent was obtained from all subjects before participating in the study.
(2) The criteria for inclusion in the healthy control group, lung cancer group and staging were as follows:
   a. The healthy control group was selected under three conditions: (1) according to the questionnaire, subjects with common chronic diseases (hypertension, diabetes, coronary heart disease), tumor treatment history and major surgery history were excluded; (2) subjects without obvious clinical symptoms in self-report were included; (3) subjects were included, whose physical examination results were within the normal range and no obvious abnormalities were found; subjects were included, whose examination results were beyond the normal range or abnormal, but were determined by the doctor to have no clinical significance. (The physical examination items included: Low-Dose Computed Tomography (LDCT), abdominal color Doppler ultrasound, 4 tumor markers, and blood pressure, as shown in the figure). The subjects who met the above three conditions were selected into the healthy control group.
   b. The lung cancer group was selected under conditions: the histological classification of lung cancer adopted the standards of the WHO Classification of Tumours of the Lung, Pleura, Thymus and Heart, 4th Edition, 2015. The stages of lung cancer standard adopted the eighth edition of the American Joint Committee on Cancer (AJCC) standard according to the TNM staging system. The final diagnosis was made by histopathological examination as a gold standard and relying on the expertise of senior clinicians.
   Exclusion criteria: under 18 years old; pregnant; duplicate samples, undiagnosed, or unqualified in quality inspection.
(3) Reagents: chromatographically pure (HPLC-grade) formic acid, acetic acid, methanol, ammonium acetate, acetonitrile, MTBE, and isopropanol were purchased from Sigma-Aldrich, USA; deionized water was prepared using an ultrapure water system from Millipore, USA.
(4) Sample preparation

100 µL of plasma was placed in 1,000 µL of pre-cooled solution (MTBE: methanol with a volume ratio of 3:1), and the extracted blood sample was mixed well by vortex to obtain the sample extract; 500 µL of solution (methanol: water with a volume ratio of 3:1) was added to the sample extract, sonicated, allowed to stand, mixed well by vortex, and centrifuged to separate layers.
- Organic phase: after the sample was layered, 500 µL of an upper phase was added into a centrifuge tube as the organic phase; after the organic phase was dried, 200 µL of (acetonitrile: isopropanol with a volume ratio of 3:1) was added and incubated at room temperature for 15 min; after incubation, the centrifuge tube was mixed well by vortex, ultrasonically treated for 5 min, and then centrifuged at room temperature for 5 min (12,000 rpm); 180 µL of an obtained supernatant from the centrifuge tube was transferred into a 2 mL glass injection vial as an organic phase substance, and detected on a machine (LC-MS).
- Aqueous phase: after the sample was layered, 400 µL of a lower phase was added into a centrifuge tube, and 1,100 µL of ice methanol was added to precipitate the protein; the centrifuge with the protein was centrifuged, 1,000 µL of an obtained supernatant was transferred to a new centrifuge tube, and dried overnight; 200 µL of water was added into the dried centrifuge tube and incubated at room temperature for 15 min; after incubation, the centrifuge tube was mixed well by vortex, ultrasonically treated for 5 min, and then centrifuged at room temperature for 5 min (12,000 rpm); 180 µL of an obtained supernatant from the centrifuge tube was transferred into a 2 mL glass injection vial as an aqueous phase substance, and detected on a machine (LC-MS).

### (5) Detection of small molecule metabolites:

Waters ACQUTTY UPLC^{®} BEH C8 1.7 µm 2.1*100 mm column was used for organic phase detection, while Waters ACQUTTY UPLC^{®} HSS T3 1.8 µm 2.1*100 mm column was used for aqueous phase detection to separate small molecules. And LC and MS instruments used ACQUITY UPLC I-Class LC system (Waters) and Q-Exactive MS system (Thermo Fisher Scientific), respectively.

The mobile phase parameters corresponding to organic phase and aqueous phase substances are as follows:
Organic phase substance: mobile phase A was an aqueous solution containing 0.1% (w/v) acetic acid and 0.1% (w/v) ammonium acetate; mobile phase B was an acetonitrile-isopropanol (7:3, v/v) solution containing 0.1% (w/v) acetic acid and 1% (w/v) ammonium acetate; elution gradient for separation was as follows: 0-12 min for 55%-89% (v/v) mobile phase B, 12-19.5 min for 100% (v/v) mobile phase B.

Aqueous phase substance: mobile phase A was an aqueous solution containing 0.1% (w/v) formic acid; mobile phase B was an acetonitrile solution containing 0.1% (w/v) formic acid. The elution gradient for separation was as follows: 0-13 min for 1%-70% (v/v) mobile phase B, 13-18 min for 99% (v/v) mobile phase B.

The MS parameters were as follows:
MS data were acquired in Full MS and Full MS/dd-MS2 modes (each with positive and negative modes). The parameters used by Q Exactive were as follows: in Full MS mode, the resolution was 70,000, the scan range was 100 to 1,500 m/z, the AGC was 3E+6, and the Maximum IT was 200 ms; in Full MS/dd-MS2 mode, the resolution of the secondary MS was 17,500, the quadrupole rod window was 1.5 m/z, the AGC was 1E+5, the maximum ion injection time was 50 ms, and the HCD relative collision energy was 30 eV.

The examples of the present disclosure had significant advantages in metabolite extraction:
The example could extract metabolites from only 100 µL of plasma. This process included processing organic phase and aqueous phase substances, using efficient stratification technology to separate the two substances at the same time and test them separately, thus finally obtaining detection data of all metabolites in the organic phase and aqueous phase. This unique process was called "all-in-one extraction", which was also one of the significant features of the examples of the present disclosure in terms of metabolite extraction. Compared with the traditional double extraction method, this method was efficient and convenient, providing a more convenient way for metabolite analysis. In addition, the method could also improve a stability of the detection results to a certain extent and ensure a reliability of the data.

(6) Metabolomics data processing: detection peaks were extracted from all MS data, noises were removed using baseline correction, and original signal peaks were retained; the original data were converted into central discrete data; the peaks in a single sample were compared with the retention time in the chromatogram for matching; the isotope peaks in data set was further removed to obtain the final MS matrix data; in order to reduce the difference in metabolite concentrations between samples and make the data distribution more symmetrical, retaining data was normalized by Normalization Autoencoder (NormAE).

(7) Identification of metabolites: public databases such as the Human Metabolite Database (HMDB; www.hmdb.ca), the Metabolomics Database (Metlin; https://metlin.scripps.edu), and the MS Database (http://www.massbank.jp/), as well as the primary and secondary chromatograms and MS spectra of the standard separated on the same chromatographic column were used; when the retention time difference was within 0.2 min and the mass-to-charge ratio was less than 10 ppm, the metabolites were matched and identified with the database and the standards..

(8) In the metabolomics data analysis, this example conducted the following five steps:
Step one, the data from the two centers were classified into a modeling set (Table 1) and a validation set (Table 2). The samples used in the modeling set and the validation set were all actual samples collected, and the modeling set samples and the validation set samples were different samples.
Step two, metabolites with significant differences between a healthy control group (HC) and a lung cancer group (LC) were selected through the modeling group samples to build a lung cancer diagnosis model and identify potential metabolic markers for lung cancer diagnosis.
Step three, lung cancer patients were divided into early stage (0+I+II) and advanced stage (III+IV) according to the stage of lung cancer (Table 1).

**Table 1. Healthy control and lung cancer grouping information of modeling set**

| | Healthy Control | Lung cancer stage 0 | Lung cancer stage I | Lung cancer stage II | Lung cancer stage III | Lung cancer stage IV |
|---|---|---|---|---|---|---|
| Healthy control and stages of lung cancer | HC | 0 | I | II | III | IV |
| Number | 236 | 10 | 290 | 40 | 116 | 123 |
| Healthy control and lung cancer | HC | Lung cancer (LC) | | | | |
| Healthy control and early and | HC | Early stage lung cancer (LC0+I+II) | | | Advanced stage lung cancer (LCIII+IV) | |
| advanced stages of lung cancer | | | | | | |

Linear correlation analysis was conducted using the above metabolic markers to calculate the Pearson correlation coefficient to evaluate the strength of the linear correlation between the two variables.

Step four, metabolites with a Pearson correlation coefficient |R| of greater than 0.3 were selected for multivariate and multi-class classification ROC analysis to evaluate their performance in classifying stages of lung cancer.

Step five, the data from the validation set (Table 2) were used to independently validate the above metabolic markers in the diagnosis and clinical staging of lung cancer.

**Table 2 Healthy control and lung cancer grouping information of validation set**

| Healthy control and stages of lung cancer | Healthy Control | Lung cancer stage 0/I | Lung cancer stage II | Lung cancer stage III | Lung cancer stage IV |
|---|---|---|---|---|---|
| Number | 55 | 65 | 10 | 26 | 30 |
| Healthy control and lung cancer | HC | Lung cancer (LC) | | | |
| Healthy control and early and advanced stages of lung cancer | HC | Early stage lung cancer (LC0+I+II) | | Advanced stage lung cancer (LCIII+IV) | |

### 2. Result analysis

### (1) Screening of metabolic markers for lung cancer diagnosis

By matching with the database and standards, a total of 404 metabolites were successfully identified in this example. In order to screen out the important metabolites that could effectively distinguish the healthy control group (HC) and lung cancer group (LC) of the modeling group from these 404 metabolites, the ROC curve analysis method was adopted.

The ROC curve was a method to study the relationship between sensitivity and specificity of model. With sensitivity as an ordinate and 1-specificity as an abscissa, the AUC of ROC curve could be used as an indicator to determine the distinguishing ability of metabolites. The AUC value, ranged from 0 to 1, reflected the ability to distinguish between positive and negative samples of model. The closer the AUC value was to 1, the better the performance of model in the classification task was and the better it could distinguish between positive and negative examples; an AUC value close to or less than 0.5 meant that the performance of model was close to random guessing, indicating that the model showed poor distinguishing ability and accuracy.

In this example, univariate ROC curve analysis was conducted for each of the 404 metabolites. 24 metabolites with an AUC value of greater than 0.70 in the analysis results were retained, and the P values of a T-test between the healthy control group (HC) and lung cancer patients (LC) for the 24 metabolites were all less than 0.05.

### (2) Screening of metabolic marker for stages of lung cancer

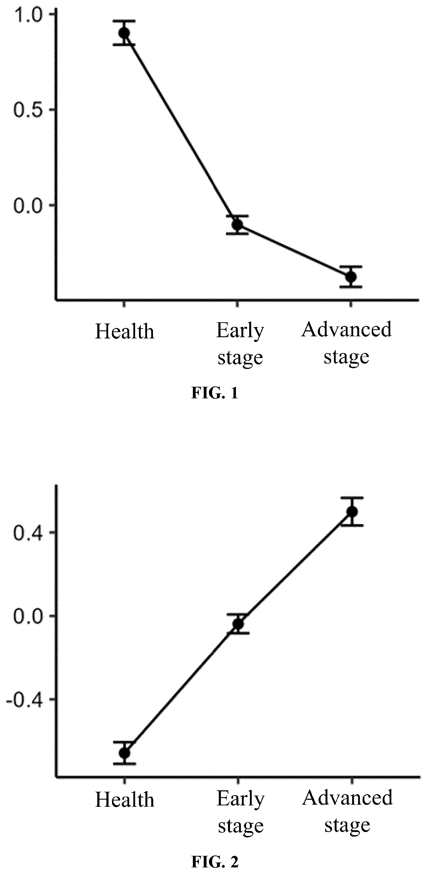

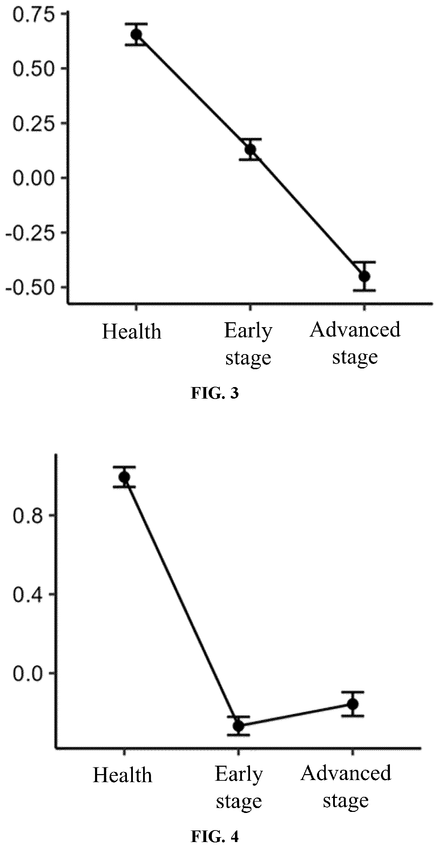

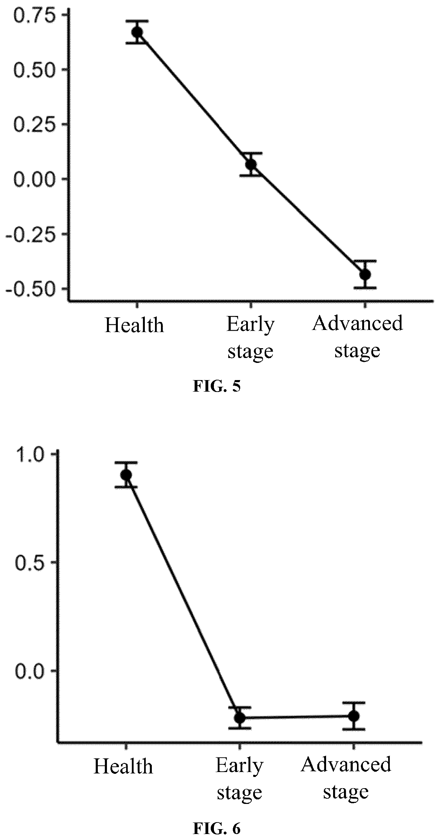

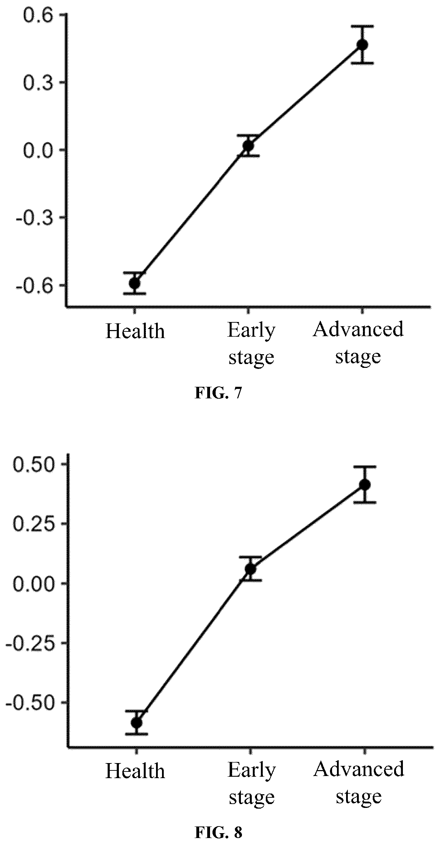

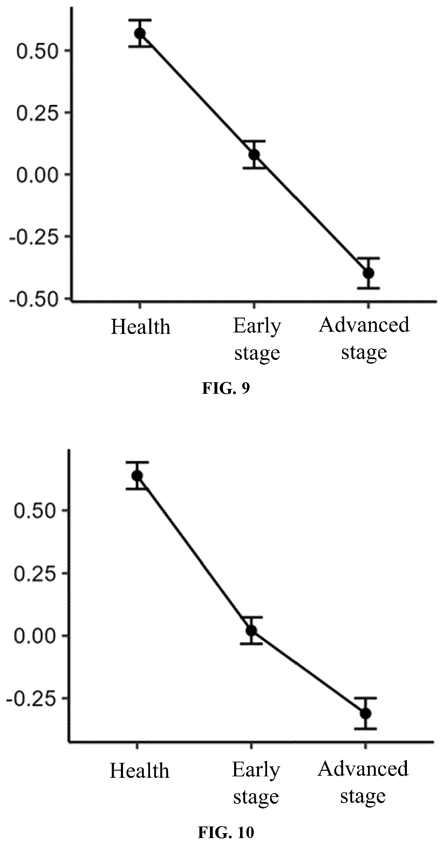

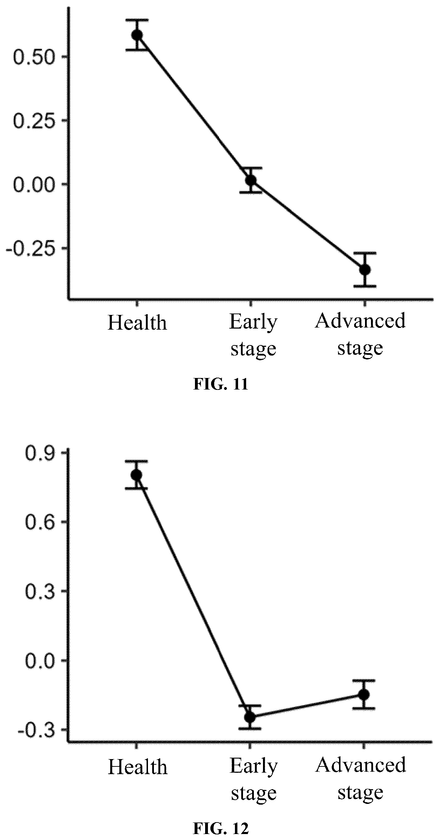

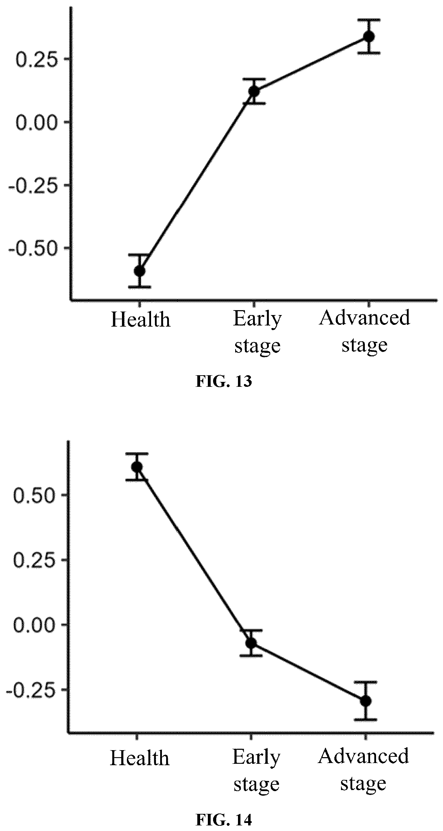

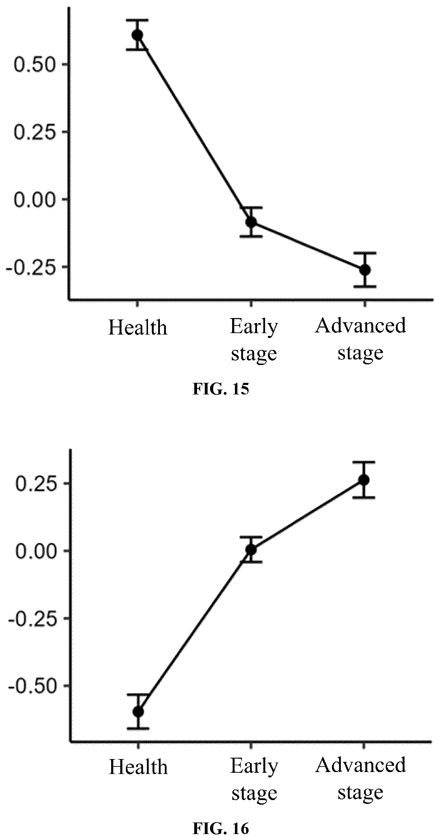

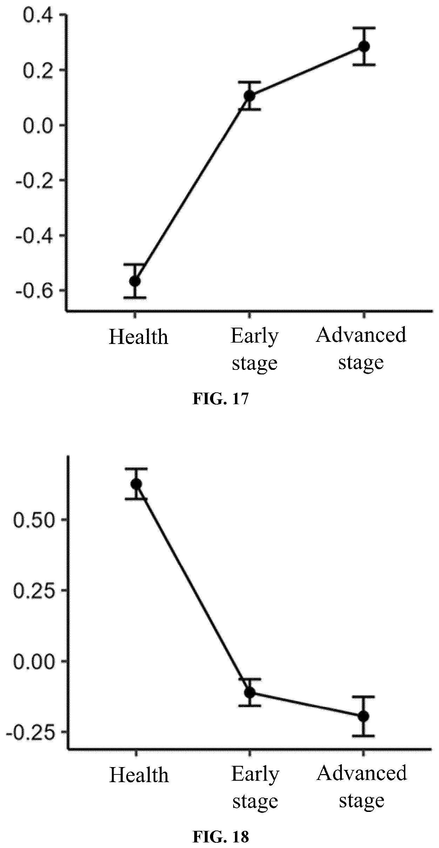

According to the stage of lung cancer, this example re-divided the cancer patients in the modeling group into early stage (0+I+II) and advanced stage (III+IV) (Table 1), and then conducted linear correlation analysis on 24 significantly differential metabolic markers for lung cancer diagnosis, including the correlation between healthy control, early stage lung cancer, and advanced stage lung cancer. 19 lung cancer metabolic markers with correlation coefficients |R|>0.3 were selected through analysis (FIG. 1 to FIG. 19 and Table 3). FIG. 1 to FIG. 19 corresponded to the metabolic markers numbered 1 to 19 in Table 3, respectively.

### (3) ROC analysis of lung cancer diagnosis and stages of lung cancer

Different from considering the performance of a single metabolite alone, multivariate ROC analysis considered the interrelationships among multiple metabolites, thus providing a more comprehensive and accurate assessment of lung cancer detection capability. This approach made full use of the correlation between metabolites and could more effectively capture the combination of disease-related markers.

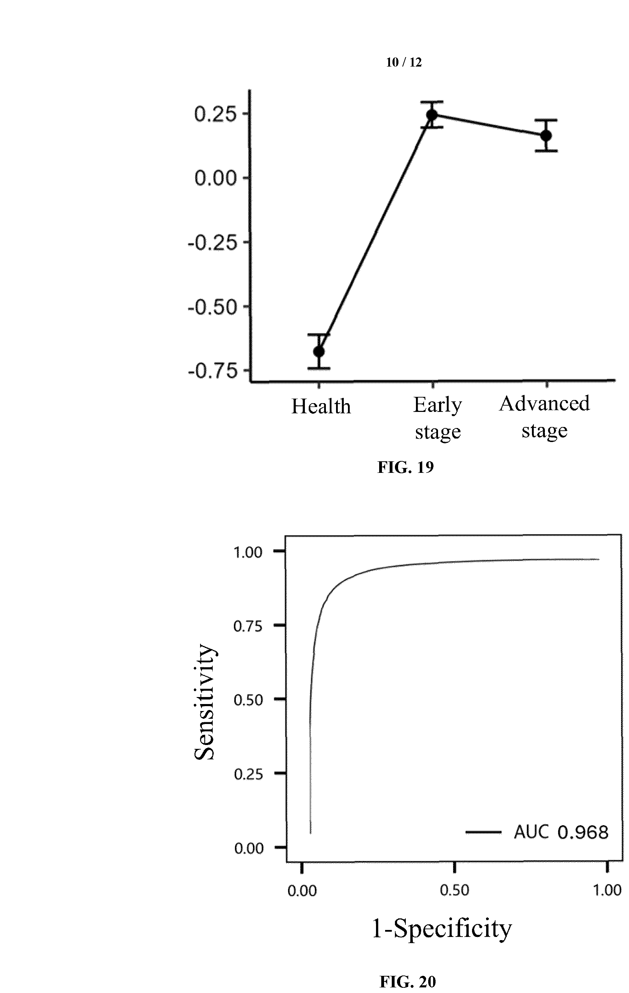

In this example, a support vector machine (SVM) algorithm in machine learning was used to learn two-dimensional matrix data, 3/4 of the sample data of the healthy control group and lung cancer group of the modeling set were randomly used as a training set, while the remaining 1/4 was used as a test set for learning and iterated 2,000 times with SVM random cycles. A lung cancer diagnosis model based on 19 significantly different metabolic markers was constructed by statistically calculating the average accuracy of the final model. Based on the selected 19 significantly different metabolites, multivariate ROC analysis was conducted on the test set to evaluate the effect of the combined application of these metabolites in lung cancer diagnosis. The classification model evaluation indicator was the AUC value in the ROC analysis. The results showed that the AUC value of the ROC multivariate analysis was 0.968 (FIG. 20), indicating that the metabolites selected in this example had high accuracy and distinguishing ability in the lung cancer diagnosis task.

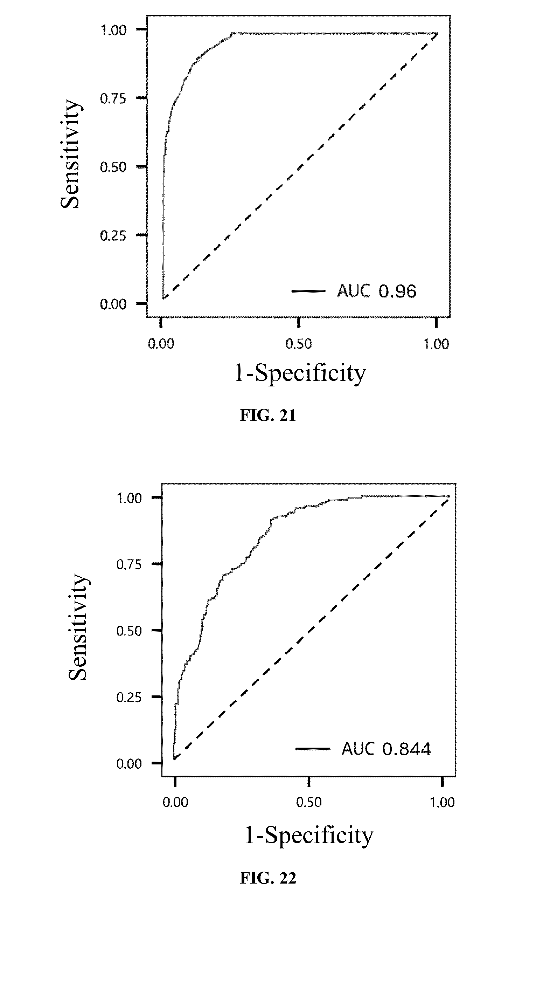

Subsequently, multivariate and multi-class classification ROC curve analysis was conducted according to the stages of lung cancer. Through multivariate ROC analysis and machine learning, the training set result was AUC=0.96 (FIG. 21), and the test set result was AUC=0.844 (FIG. 22), indicating that the selected metabolic markers could efficiently achieve early diagnosis of lung cancer and prediction of cancer clinical staging at one time.

### (4) Independent verification using validation set data

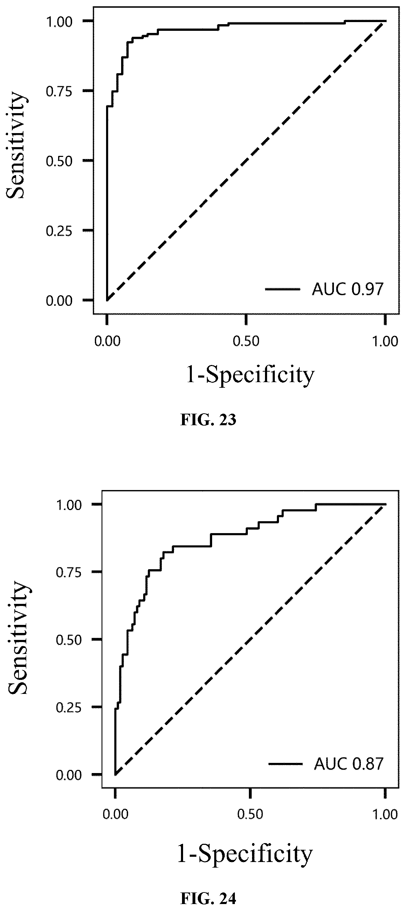

The validation set data were used to diagnose HC vs LC and HC and LC 0+I+II, LC III+IV. The results showed that the AUC value of the multivariate ROC analysis of HC vs LC was 0.97 (FIG. 23). For HC and LC 0+I+II, LC III+IV, the AUC value of the multivariate and multi-class classification ROC analysis was 0.87 (FIG. 24). These results indicated that the model had high performance to diagnose under different classification situations, providing potential clinical application value for early diagnosis of lung cancer and prediction of clinical staging of lung cancer.

**Table 3 R absolute values and P values of linear correlations among 19 metabolic markers of lung cancer**

| Number | Metabolic markers - English | Metabolic markers | \|R\| | *P*-value |
|---|---|---|---|---|
| 1 | Dehydroisoandrosterone-3-sulfate | Dehydroisoandrosterone-3-sulfate | 0.483 | 3.47E-48 |
| 2 | 4-Hydroxy-1-(3-pyridyl)-1-butanone (4-HPB) | 4-Hydroxy-1-(3-pyridyl)-1-butanone (4-HPB) | 0.453 | 6.09E-42 |
| 3 | L-Leucyl-L-leucine | L-Leucyl-L-leucine | 0.441 | 1.34E-39 |
| 4 | L-Octanoylcarnitine | L-Octanoylcarnitine | 0.435 | 1.99E-38 |
| 5 | Phosphatidylcholine 34:3e (PC 34:3e) | Phosphatidylcholine 34:3e (PC 34:3e) | 0.43 | 1.47E-37 |
| 6 | Acyl carnitine 10:0 (AcCa 10:0) | Acyl carnitine 10:0 (AcCa 10:0) | 0.415 | 7.35E-35 |
| 7 | N-Formyl-L-methionine | N-Formyl-L-methionine | 0.395 | 2.24E-31 |
| 8 | 5'-Methylthioadenosine | 5'-Methylthioadenosine | 0.379 | 7.35E-29 |
| 9 | Phosphatidylcholine 36:3e (PC 36:3e) | Phosphatidylcholine 36:3e (PC 36:3e) | 0.374 | 3.72E-28 |
| 10 | Fatty acid 22:6 (FA 22:6) | Fatty acid 22:6 (FA 22:6) | 0.369 | 2.50E-27 |
| 11 | L-Tryptophyl-L-phenylalanine | L-Tryptophyl-L-phenylalanine | 0.358 | 8.86E-26 |
| 12 | Acyl carnitine 10:1(AcCa 10:1) | Acyl carnitine 10:1 (AcCa 10:1) | 0.357 | 1.55E-25 |
| 13 | Hypoxanthine | Hypoxanthine | 0.35 | 1.29E-24 |
| 14 | L-Alanyl-L-aspartic acid | L-Alanyl-L-aspartic acid | 0.347 | 3.73E-24 |
| 15 | Iminodiacetic acid | Iminodiacetic acid | 0.335 | 1.27E-22 |
| 16 | Betaine | Betaine | 0.333 | 2.65E-22 |
| 17 | Choline | Choline | 0.323 | 4.62E-21 |
| 18 | O-Palmitoyl-L-carnitine | O-Palmitoyl-L-carnitine | 0.321 | 8.83E-21 |
| 19 | L-Glutamic acid | L-Glutamic acid | 0.314 | 7.35E-20 |

In general, the lung cancer metabolic markers screened in this example have multiple advantages, including non-invasiveness, high prediction accuracy, rapid acquisition of test results, and certain advantages in early diagnosis and clinical staging prediction. These advantages help monitor tumor growth and changes in treatment efficacy, improve the chance of early diagnosis, and reduce the risk of death from late prognosis.

The technical features of the foregoing examples can be employed in arbitrary combinations. For brevity of description, not all possible combinations of the technical features of the foregoing examples are described. However, the combinations of the technical features should be construed as falling within the scope described in this specification as long as there is no contradiction in the combinations.

The above examples are merely illustrative of several embodiments of the present disclosure, and the description thereof is more specific and detailed, but is not to be construed as a limitation to the protection scope of the present disclosure. It should be noted that those of ordinary skill in the art can further make variations and improvements without departing from the concept of the present disclosure. These variations and improvements all fall within the protection scope of the present disclosure. In addition, it should be understood that after reading the above teachings of the present disclosure, various changes and modifications may be made on the present disclosure by a person skilled in the art, and these equivalent forms also fall within the scope defined by the present disclosure. Therefore, all technical solutions that can be obtained by a person skilled in the artthrough logical analysis, deduction, or limited experiments according to the technical proposal of the present disclosureshould fall within the protection scope defined by the claims. The protection scope claimed in the present disclosure shall be subject to the content of the claims, and the description and drawings may be intended to interpret the content of the claims.

## Claims

1. Use of metabolic markers for diagnosing stages of lung cancer and one or more of a detection reagent of the metabolic markers for diagnosing stages of lung cancer in preparation of a kit for diagnosing the stages of lung cancer; wherein
the metabolic markers for diagnosing stages of lung cancer are selected from the group consisting of dehydroisoandrosterone-3-sulfate, 4-hydroxy-1-(3-pyridyl)-1-butanone, L-leucyl-L-leucine, L-octanoylcarnitine, phosphatidylcholine 34:3e, acyl carnitine 10:0, N-formyl-L-methionine, 5'-methylthioadenosine, phosphatidylcholine 36:3e, fatty acid 22:6, L-tryptophyl-L-phenylalanine, acyl carnitine 10:1, hypoxanthine, L-alanyl-L-aspartic acid, iminodiacetic acid, betaine, choline, O-palmitoyl-L-carnitine, and L-glutamic acid.

2. The use according to claim 1, wherein the detection reagent detects the metabolic markers for diagnosing stages of lung cancer through liquid chromatography-mass spectrometry (LC-MS).

3. The use according to claim 1 or 2, wherein a sample detected by the detection reagent comprises a blood sample.

4. The use according to claim 3, wherein the blood sample is selected from the group consisting of a plasma and a serum.

5. The use according to any one of claims 1 to 4, wherein the detection reagent detects the metabolic markers for diagnosing stages of lung cancer through the LC-MS comprises the following steps:
separating an organic phase and an aqueous phase from the plasma; and
detecting the organic phase and the aqueous phase through the LC-MS respectively to detect the metabolic markers for diagnosing stages of lung cancer.

6. The use according to claim 5, wherein liquid chromatography (LC) for detecting the organic phase comprises:
a C8 chromatographic column as a stationary phase;
a mobile phase comprising a mobile phase A and a mobile phase B, wherein the mobile phase A is an aqueous solution containing approximately 0.08% weight per volume (w/v) to approximately 0.12% (w/v) of acetic acid and approximately 0.08% (w/v) to approximately 0.12% (w/v) of ammonium acetate; and the mobile phase B is a mixed solution of acetonitrile and isopropanol at a volume ratio of approximately (6.5-7.5):(2.5-3.5) containing approximately 0.08% (w/v) to approximately 0.12% (w/v) of the acetic acid and approximately 0.8% (w/v) to approximately 1.2% (w/v) of the ammonium acetate; and
an elution method comprises gradient elution, and a procedure of the gradient elution comprises:
at approximately 0 min to approximately 12 min, the mobile phase B has a volume percentage increasing from approximately 55% to approximately 89%; and
at approximately 12 min to approximately 19.5 min, the mobile phase B has a volume percentage increasing from approximately 89% to approximately 100%.

7. The use according to any one of claims 5 to 6, wherein LC for detecting the aqueous phase comprises:
a T3 chromatographic column as a stationary phase;
a mobile phase comprising a mobile phase A and a mobile phase B, wherein the mobile phase A is an aqueous solution containing approximately 0.08% (w/v) to approximately 0.12% (w/v) of formic acid; and the mobile phase B is an acetonitrile solution containing approximately 0.08% (w/v) to approximately 0.12% (w/v) of the formic acid; and
an elution method comprises gradient elution, and a procedure of the gradient elution comprises:
at approximately 0 min to approximately 13 min, the mobile phase B has a volume percentage increasing from approximately 1% to approximately 70%; and
at approximately 13 min to approximately 18 min, the mobile phase B has a volume percentage increasing from approximately 70% to approximately 99%.

8. The use according to any one of claims 5 to 7, wherein mass spectrometry (MS) comprises:
conducting acquisition in two modes of a Full MS mode and a Full MS/dd-MS2 mode, wherein the two modes each comprise positive and negative modes;
the Full MS mode has a resolution of approximately 35,000 to approximately 70,000, a scan range of approximately 100 m/z to approximately 1,500 m/z, an automatic gain control (AGC) of approximately 3E+6, and a Maximum injection time (Maximum IT) of approximately 150 ms to approximately 250 ms; and
the Full MS/dd-MS2 mode has a secondary MS resolution of approximately 17,500 to approximately 35,000, a quadrupole rod window of approximately 1.2 m/z to approximately 1.6 m/z, an AGC of 1E+5, a maximum injection time of ion of approximately 45 ms to approximately 55 ms, and a high-energy collision dissociation (HCD) relative collision energy of approximately 10 eV to approximately 45 eV.

9. The use according to any one of claims 5 to 8, wherein the separating the organic phase and the aqueous phase from the plasma comprises:
extracting the plasma with a solvent 1 to obtain an extract; wherein the solvent 1 comprises methyl tert-butyl ether (MTBE) and methanol; and
extracting the extract with a solvent 2, and conducting layering to collect an obtained upper layer to obtain the organic phase and collect an obtained lower layer to obtain the aqueous phase; wherein the solvent 2 comprises methanol and water.

10. A kit for diagnosing stages of lung cancer, comprising the metabolic markers for diagnosing stages of lung cancer and one or more of a detection reagent of the metabolic markers for diagnosing stages of lung cancer according to any one of claims 1 to 9.
